# EUROPEAN PATENT SPECIFICATION

(11) **EP 2 021 463 B1**
(45) Date of publication and mention of the grant of the patent: **23.11.2016**
(21) Application number: 07861311.4
(22) Date of filing: 21.05.2007
(51) Int. Cl.: C12N 5/0781, C07K 16/00

(54) **CULTURE METHOD FOR OBTAINING A CLONAL POPULATION OF ANTIGEN-SPECIFIC B CELLS**
ANBAUVERFAHREN ZUM ERHALTEN EINER KLONPOPULATION VON ANTIGENSPEZIFISCHEN B-ZELLEN
PROCÉDÉ DE CULTURE PERMETTANT D'OBTENIR UNE POPULATION CLONÉE DE LYMPHOCYTES B SPÉCIFIQUES D'ANTIGÈNE

(30) Priority: 19.05.2006 US 801412 P
(43) Date of publication of application: 11.02.2009
(73) Proprietor: ALDER BIOPHARMACEUTICALS, INC., Bothell, WA 98011 (US)
(72) Inventor: GARCIA, Leon, Woodinville, WA 98072 (US); JENSEN, Anne, Elisabeth, Carvalho, Bothell, WA 98012 (US); OJALA, Ethan, Lynwood, WA 98030 (US); LATHAM, John, Seattle, WA 98119 (US)
(74) Representative: Boult Wade Tennant
(86) International application number: PCT/US2007/011980
(87) International publication number: WO 2008/045140

(56) References cited:
- US-A1- 2006 040 363
- US-A1- 2006 051 348
- US-B1- 6 984 383
- SIMONSSON LAGERKVIST A C ET AL: "SINGLE, ANTIGEN-SPECIFIC B CELLS USED TO GENERATE FAB FRAGMENTS USING CD40-MEDIATED AMPLIFICATION OR DIRECT PCR CLONING" BIOTECHNIQUES, INFORMA LIFE SCIENCES PUBLISHING, WESTBOROUGH, MA, US, vol. 18, no. 5, 1 May 1995 (1995-05-01), pages 862,864-869, XP000572659 ISSN: 0736-6205
- DE WILDT R M ET AL: "Isolation and characterization of single anti-U1A-specific B cells from autoimmune patients." ANNALS OF THE NEW YORK ACADEMY OF SCIENCES 5 APR 1997, vol. 815, 5 April 1997 (1997-04-05), pages 440-442, XP002536692 ISSN: 0077-8923
- WEITKAMP JORN-HENDRIK ET AL: "Generation of recombinant human monoclonal antibodies to rotavirus from single antigen-specific B cells selected with fluorescent virus-like particles." JOURNAL OF IMMUNOLOGICAL METHODS, vol. 275, no. 1-2, 1 April 2003 (2003-04-01), pages 223-237, XP004416763 ISSN: 0022-1759
- DOHMEN S E ET AL: "Production of recombinant Ig molecules from antigen-selected single B cells and restricted usage of Ig-gene segments by anti-D antibodies" JOURNAL OF IMMUNOLOGICAL METHODS, ELSEVIER SCIENCE PUBLISHERS B.V.,AMSTERDAM, NL, vol. 298, no. 1-2, 1 March 2005 (2005-03-01), pages 9-20, XP004853233 ISSN: 0022-1759
- KODITUWAKKU ET AL.: 'Isolation of antigen-specific B cells' IMMUNOLOGY AN CELL BIOLOGY vol. 81, no. 3, June 2003, pages 163 - 170
- STEENBAKKERS ET AL.: 'Efficient generation of monoclonal antibodies from preselected antigen-specific B cells' MOLECULAR BIOLOGY REPORTS vol. 19, no. 2, March 1994, pages 125 - 134, XP000673961

## Description

This application claims the benefit of U.S. Provisional Patent Application Ser. No. 60/801,412 filed May 19, 2006.

### FIELD OF INVENTION

The present invention relates to culture methods for obtaining a clonal population of antigen-specific cells.

### BACKGROUND OF THE INVENTION

Methods for culturing and identifying B cells that produce antibodies specific to a desired antigen are well known in the art. Such B cells are useful for the recovery of antigen-specific antibodies and for the recovery of nucleic acid sequences encoding such antibodies. Such B cells can also be used in antigen-specific functional assays.

Antibodies are used by the immune system to identify foreign antigens such as toxins, bacteria, and viruses. Each antibody binds to a specific epitope of the antigen. The antibody's ability to recognize and bind to a specific epitope makes the antibody a useful therapeutic and diagnostic tool. In addition to their immunological role, antibodies can be produced to recognize virtually any substance, including other proteins, such as growth factors, hormones, and enzymes.

Methods of producing monoclonal antibodies include somatic cell hybridization whereby an animal is immunized with an antigen to induce an immunological response, the animal's B cells are harvested and fused to an immortal cell line to form hybridomas, and the hybridomas are screened to identify a clone with antigen specificity. But the low frequency of antigen-specific B cells makes it difficult to isolate an antigen-specific clone. The frequency of desirable candidates is further reduced when seeking antigen-specific B cells that also exhibit a particular epitope specificity or functional activity.

Additionally, monoclonal antibodies can be produced by cloning antibody-encoding nucleic acid sequences from a B cell that produces a monoclonal antibody specific to a desired antigen, and expressing these nucleic acid sequences or modified sequences derived therefrom in a suitable recombinant expression system such a mammalian cells or bacterial expression systems. Such methods are preferred over hybridoma techniques as they allow for production of a limitless supply of monoclonal antibodies having a desired antigen specificity while also allowing for such antibody sequences to be modified such as by humanization or chimerisation.

The present invention provides culture methods for isolating a clonal population of antigen-specific cells. A clonal population of B cells is potentially useful in B cell functional assays as well as for the recovery of antigen-specific monoclonal antibodies and for the recovery nucleic acid sequences that encode such antibodies.

Simonsson Lagerkvist *et al* disclose a procedure for the generation of human antibody fragments directly from single B cells or B-cell clones (Biotechniques, Vol. 18, No. 5 (1995)).

De Wildt *et al* disclose the isolation and characterization of single anti-U1A-specific B cells from autoimmune patients (Annals of the New York Academy of Sciences, Vol. 815, pages 440-442 (1997)).

Dohmen *et al* disclose the production of recombinant Ig molecules from antigen-selected single B cells and restricted usage of Ig-gene segments by anti-D antibodies (Journal of Immunological Methods, Vol. 298, pages 9-20 (2005)).

### BRIEF DESCRIPTION OF THE FIGURES

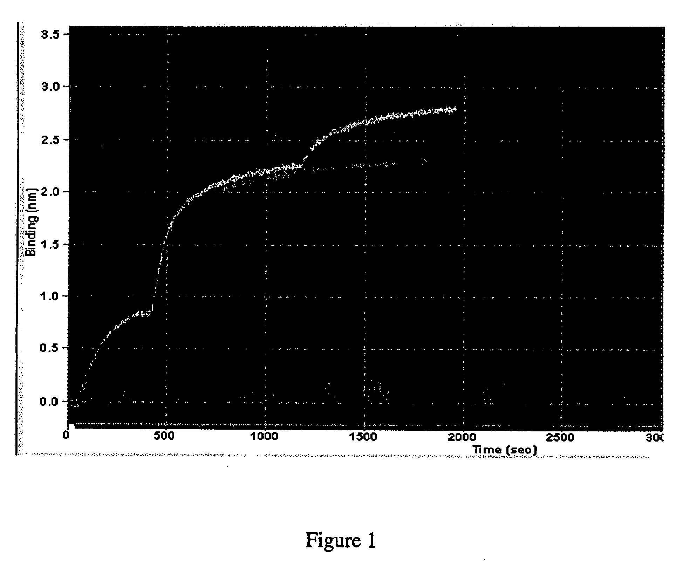

Fig. 1 shows that a variety of unique epitopes were recognized by the collection of anti-IL-6 antibodies prepared by the antibody selection protocol. Epitope variability was confirmed by antibody-IL-6 binding competition studies (ForteBio Octet).
Fig. 2 shows that a variety of unique epitopes were recognized by the collection of anti-TNF-α antibodies prepared by the antibody selection protocol. Epitope variability was confirmed by antibody-TNF-α binding competition studies (ForteBio Octet).

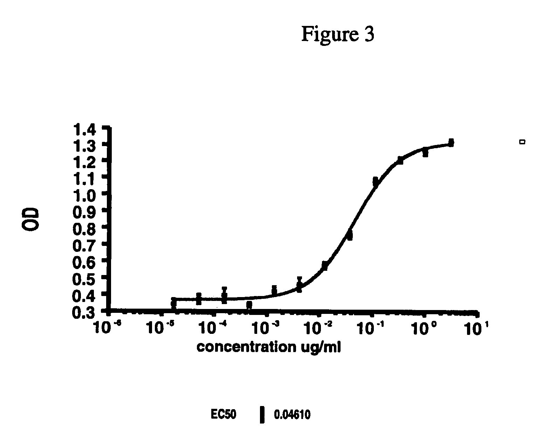

Fig. 3 depicts the binding affinity of an anti-TNF-α antibody.

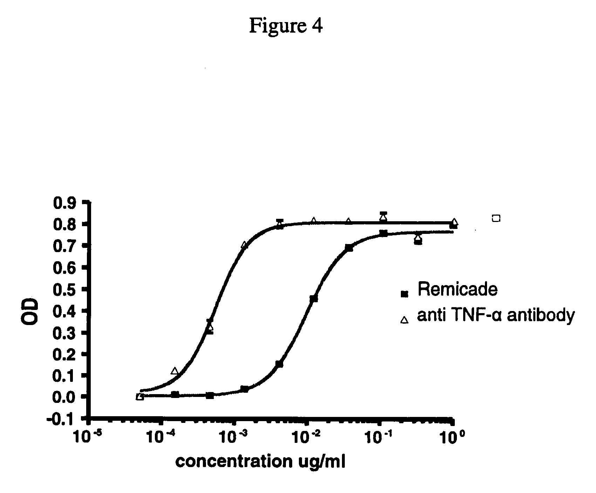

Fig. 4 depicts the comparative cytotoxicity of an anti-TNF-α antibody.

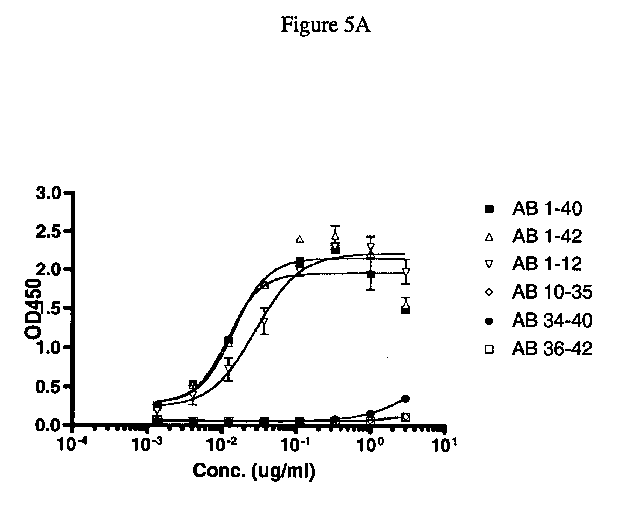

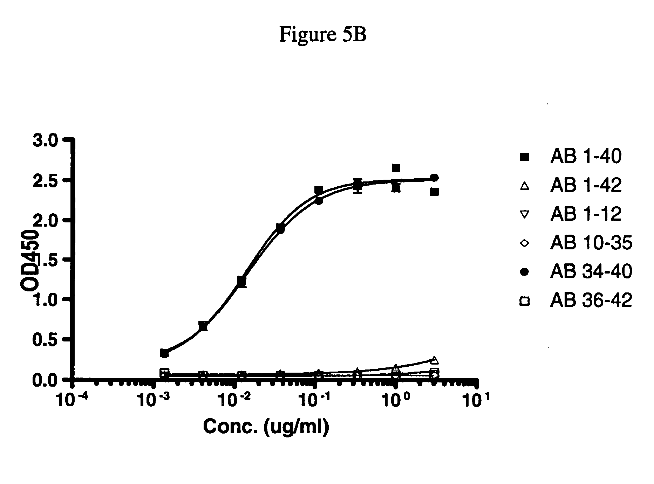

Fig. 5 depicts the epitope selectivity of anti-Aβ antibodies.
Fig. 6 demonstrates the high correlation between the IgG produced and antigen specificity for an exemplary IL-6 protocol. 9 of 11 wells showed specific IgG correlation with antigen recognition.
Fig. 7 demonstrates the high correlation between the IgG produced and antigen specificity for an exemplary huTNF-α protocol. 18 of 20 wells showed specific IgG correlation with antigen recognition.

### DETAILED DESCRIPTION OF THE INVENTION

The present invention provides a method of identifying a B cell that secretes an antigen-specific antibody, the method comprising:
(a) harvesting B cells from an immunised rabbit host;
(b) enriching the harvested rabbit B cells to increase the proportion of B cells that are specific for said antigen by affinity purification of antigen-specific B cells using an antigen directly or indirectly attached to a solid matrix or support, thereby forming an enriched B cell population;
(c) culturing one or more sub-populations of cells from said enriched rabbit B cell population under conditions that favour the survival of a single B cell to produce a clonal population in at least one culture well;
(d) determining whether said cultured sub-populations produce an antibody specific to said antigen, thereby identifying one or more antigen-positive sub-populations; and
(e) determining whether a single rabbit B cell produces an antibody specific to the antigen by a method comprising:
   (i) isolating one or more single rabbit B cells from one or more of said antigen-positive sub-populations of step (d);
   (ii) providing an immobilized antigen comprising a matrix or solid support to which said antigen has been directly or indirectly attached;
   (iii) incubating an individual rabbit B cell with said immobilized antigen;
   (iv) incubating said immobilized antigen with a fluorophore labeled secondary antibody, wherein said secondary antibody is a host-specific anti-immunoglobulin antibody that binds antibodies of the host of step (a), thereby detecting whether an antibody secreted by said individual B cell is bound to said immobilized antigen; and
   (v) identifying a B cell that secretes an antibody specific to said antigen by a fluorimetric assay that detects antibody-antigen complexes formed adjacent to the secreting cell.

Described herein are methods of isolating a clonal population of antigen-specific B cells that may be used for isolating at least one antigen-specific cell. As described and exemplified *infra,* these methods contain a series of culture and selection steps that can be used separately, in combination, sequentially, repetitively, or periodically. Preferably, these methods are used for isolating at least one antigen-specific cell, which can be used to produce a monoclonal antibody, which is specific to a desired antigen, or a nucleic acid sequence corresponding to such an antibody.

Described herein is a method comprising the steps of:
a. preparing a cell population comprising at least one antigen-specific B cell;
b. enriching the cell population, e.g., by chromatography, to form an enriched cell population comprising at least one antigen-specific B cell;
c. isolating a single B cell from the enriched B cell population; and
d. determining whether the single B cell produces an antibody specific to the antigen.

Described herein is an improvement to a method of isolating a single, antibody-producing B cell, the improvement comprising enriching a B cell population obtained from a host that has been immunized or naturally exposed to an antigen, wherein the enriching step precedes any selection steps, comprises at least one culturing step, and results in a clonal population of B cells that produces a single monoclonal antibody specific to said antigen.

Throughout this application, a "clonal population of B cells" refers to a population of B cells that only secrete a single antibody specific to a desired antigen. That is to say that these cells produce only one type of monoclonal antibody specific to the desired antigen.

In the present application, "enriching" a cell population cells means increasing the frequency of desired cells, typically antigen-specific cells, contained in a mixed cell population, e.g., a B cell-containing isolate derived from a host that is immunized against a desired antigen. Thus, an enriched cell population encompasses a cell population having a higher frequency of antigen-specific cells as a result of an enrichment step, but this population of cells may contain and produce different antibodies.

The general term "cell population" encompasses pre- and a post-enrichment cell populations, keeping in mind that when multiple enrichment steps are performed, a cell population can be both pre- and post-enrichment.

Described herein is a method:
a. harvesting a cell population from an immunized host to obtain a harvested cell population;
b. creating at least one single cell suspension from the harvested cell population;
c. enriching at least one single cell suspension to form a first enriched cell population;
d. enriching the first enriched cell population to form a second enriched cell population;
e. enriching the second enriched cell population to form a third enriched cell population; and
f. selecting an antibody produced by an antigen-specific cell of the third enriched cell population.

Each cell population may be used directly in the next step, or it can be partially or wholly frozen for long- or short- term storage or for later steps. Also, cells from a cell population can be individually suspended to yield single cell suspensions. The single cell suspension can be enriched, such that a single cell suspension serves as the pre-enrichment cell population. Then, one or more antigen-specific single cell suspensions together form the enriched cell population; the antigen-specific single cell suspensions can be grouped together, e.g., re-plated for further analysis and/or antibody production.

Described herein is a method of enriching a cell population to yield an enriched cell population having an antigen-specific cell frequency that is about 50% to about 100%, or increments therein. Preferably, the enriched cell population has an antigen-specific cell frequency greater than or equal to about 50%, 60%, 70%, 75%, 80%, 90%, 95%, 99%, or 100%.

Described herein is a method of enriching a cell population whereby the frequency of antigen-specific cells is increased by at least about 2-fold, 5-fold, 10-fold, 20-fold, 50-fold, 100-fold, or increments therein.

Throughout this application, the term "increment" is used to define a numerical value in varying degrees of precision, e.g., to the nearest 10, 1, 0.1, 0.01, etc. The increment can be rounded to any measurable degree of precision, and the increment need not be rounded to the same degree of precision on both sides of a range. For example, the range 1 to 100 or increments therein includes ranges such as 20 to 80, 5 to 50, and 0.4 to 98. When a range is open-ended, e.g., a range of less than 100, increments therein means increments between 100 and the measurable limit. For example, less than 100 or increments therein means 0 to 100 or increments therein unless the feature, e.g., temperature, is not limited by 0.

Antigen-specificity can be measured with respect to any antigen. The antigen can be any substance to which an antibody can bind including, but not limited to, peptides, proteins or fragments thereof; carbohydrates; organic and inorganic molecules; receptors produced by animal cells, bacterial cells, and viruses; enzymes; agonists and antagonists of biological pathways; hormones; and cytokines. Exemplary antigens include, but are not limited to, IL-2, IL-4, IL-6, IL-10, IL-12, IL-13, IL-18, IFN-α, IFN-γ, BAFF, CXCL13, IP-10, VEGF, EPO, EGF, HRG, and Aβ. Preferred antigens include IL-6, IL-13, TNF-α and VEGF-α. In a method utilizing more than one enrichment step, the antigen used in each enrichment step can be the same as or different from one another. Multiple enrichment steps with the same antigen may yield a large and/or diverse population of antigen-specific cells; multiple enrichment steps with different antigens may yield an enriched cell population with cross-specificity to the different antigens.

Enriching a cell population can be performed by any cell-selection means known in the art for isolating antigen-specific cells. For example, a cell population can be enriched by chromatographic techniques, e.g., Miltenyi bead or magnetic bead technology. The beads can be directly or indirectly attached to the antigen of interest. In a preferred embodiment, the method of enriching a cell population includes at least one chromatographic enrichment step.

A cell population can also be enriched by performed by any antigen-specificity assay technique known in the art, e.g., an ELISA assay or a halo assay. ELISA assays include, but are not limited to, selective antigen immobilization (e.g., biotinylated antigen capture by streptavidin, avidin, or neutravidin coated plate), non-specific antigen plate coating, and through an antigen build-up strategy (e.g., selective antigen capture followed by binding partner addition to generate a heteromeric protein-antigen complex). A halo assay comprises contacting the cells with antigen-loaded beads and labeled anti-host antibody specific to the host used to harvest the B cells. The label can be, e.g., a fluorophore. In one embodiment, at least one assay enrichment step is performed on at least one single cell suspension. In another embodiment, the method of enriching a cell population includes at least one chromatographic enrichment step and at least one assay enrichment step.

Methods of "enriching" a cell population by size or density are known in the art. See, e.g., U.S. Patent 5,627,052. These steps can be used in the present method in addition to enriching the cell population by antigen-specificity.

The cell populations of the methods described herein contain at least one cell capable of recognizing an antigen. Antigen-recognizing cells include, but are not limited to, B cells, plasma cells, and progeny thereof. In one embodiment, the methods described herein provide a clonal cell population containing a single type of antigen-specific B-cell, i.e., the cell population produces a single monoclonal antibody specific to a desired antigen.

In such embodiment, it is believed that the clonal antigen-specific population of B cells consists predominantly of antigen-specific, antibody-secreting cells, which are obtained by the novel culture and selection protocol provided herein. Described herein are methods for obtaining an enriched cell population containing at least one antigen-specific, antibody-secreting cell. Described herein are methods for obtaining an enriched cell population containing about 50% to about 100%, or increments therein, or greater than or equal to about 60%, 70%, 80%, 90%, or 100% of antigen-specific, antibody-secreting cells.

Described herein is a method of isolating a single B cell by enriching a cell population obtained from a host before any selection steps, e.g., selecting a particular B cell from a cell population and/or selecting an antibody produced by a particular cell. The enrichment step can be performed as one, two, three, or more steps. In one embodiment, a single B cell is isolated from an enriched cell population before confirming whether the single B cell secretes an antibody with antigen-specificity and/or a desired property.

In one embodiment, a method of enriching a cell population is used in a method for antibody production and/or selection. Described herein is a method comprising enriching a cell population before selecting an antibody. The method can include the steps of: preparing a cell population comprising at least one antigen-specific cell, enriching the cell population by isolating at least one antigen-specific cell to form an enriched cell population, and inducing antibody production from at least one antigen-specific cell. In a preferred embodiment, the enriched cell population contains more than one antigen-specific cell. In one embodiment, each antigen-specific cell of the enriched population is cultured under conditions that yield a clonal antigen-specific B cell population before isolating an antibody producing cell therefrom and/or producing an antibody using said B cell, or a nucleic acid sequence corresponding to such an antibody. In contrast to prior techniques where antibodies are produced from a cell population with a low frequency of antigen-specific cells, the present invention allows antibody selection from among a high frequency of antigen-specific cells. Because an enrichment step is used prior to antibody selection, the majority of the cells, preferably virtually all of the cells, used for antibody production are antigen-specific. By producing antibodies from a population of cells with an increased frequency of antigen specificity, the quantity and variety of antibodies are increased.

In the antibody selection methods described herein, an antibody is preferably selected after an enrichment step and a culture step that results in a clonal population of antigen-specific B cells. The methods can further comprise a step of sequencing a selected antibody or portions thereof from one or more isolated, antigen-specific cells. Any method known in the art for sequencing can be employed and can include sequencing the heavy chain, light chain, variable region(s), and/or complementarity determining region(s) (CDR).

In addition to the enrichment step, the method for antibody selection can also include one or more steps of screening a cell population for antibody functionality. For example, the desired antibodies may have specific structural features, such as binding to a particular epitope or mimicry of a particular structure; antagonist or agonist activity; or neutralizing activity, e.g., inhibiting binding between the antigen and a ligand. In one embodiment, the antibody functionality screen is ligand-dependent. Screening for antibody functionality includes, but is not limited to, an *in vitro* protein-protein interaction assay that recreates the natural interaction of the antigen ligand with recombinant receptor protein; and a cell-based response that is ligand dependent and easily monitored (e.g., proliferation response). In one embodiment, the method for antibody selection includes a step of screening the cell population for antibody functionality by measuring the inhibitory concentration (IC₅₀). In one embodiment, at least one of the isolated, antigen-specific cells produces an antibody having an IC₅₀ of less than about 100, 50, 30, 25, 10 µg/mL, or increments therein.

In addition to the enrichment step, the method for antibody selection can also include one or more steps of screening a cell population for antibody binding strength. Antibody binding strength can be measured by any method known in the art (e.g., Biacore). In one embodiment, at least one of the isolated, antigen-specific cells produces an antibody having a high antigen affinity, e.g., a dissociation constant (K_{d}) of less than about 5x10⁻¹⁰ M⁻¹, preferably about 1x10⁻¹³ to 5x10⁻¹⁰, 1x10⁻¹² to 1x10⁻¹⁰, 1x10⁻¹² to 7.5x10⁻¹¹, 1x10⁻¹¹ to 2x10⁻¹¹, about 1.5x10⁻¹¹ or less, or increments therein. In this embodiment, the antibodies are said to be affinity mature. In a preferred embodiment, the affinity of the antibodies is comparable to or higher than the affinity of any one of Panorex® (edrecolomab), Rituxan® (rituximab), Herceptin® (traztuzumab), Mylotarg® (gentuzumab), Campath® (alemtuzumab), Zevalin™ (ibritumomab), Erbitux™ (cetuximab), Avastin™ (bevicizumab), Raptiva™ (efalizumab), Remicade® (infliximab), Humira™ (adalimumab), and Xolair™ (omalizumab). Preferably, the affinity of the antibodies is comparable to or higher than the affinity of Humira™. The affinity of an antibody can also be increased by known affinity maturation techniques. In one embodiment, at least one cell population is screened for both antibody functionality and antibody binding strength.

In addition to the enrichment step, the method for antibody selection can also include one or more steps of screening a cell population for antibody sequence homology, especially human homology. In another embodiment, at least one of the isolated, antigen-specific cells produces an antibody that has a homology to a human antibody of about 50% to about 100%, or increments therein, or greater than about 60%, 70%, 80%, 85%, 90%, or 95% homologous. The antibodies can be humanized to increase the homology to a human sequence by techniques known in the art such as CDR grafting or selectivity determining residue grafting (SDR).

Also described herein are the antibodies themselves according to any of the embodiments described above in terms of IC₅₀, K_{d}, and/or homology.

The inventive B cell selection protocol disclosed herein has a number of intrinsic advantages versus other methods for obtaining antibody-secreting B cells and monoclonal antibodies specific to desired target antigens. These advantages include, but are not restricted to, the following:
First, it has been found that when these selection procedures are utilized with a desired antigen such as IL-6 or TNF-α, the methods reproducibly result in antigen-specific B cells capable of generating what appears to be a substantially comprehensive complement of antibodies, i.e., antibodies that bind to the various different epitopes of the antigen. Without being bound by theory, it is hypothesized that the comprehensive complement is attributable to the antigen enrichment step that is performed prior to initial B cell recovery. Moreover, this advantage allows for the isolation and selection of antibodies with different properties as these properties may vary depending on the epitopic specificity of the particular antibody.
Second, it has been found that the inventive B cell selection protocol reproducibly yields a clonal B cell culture containing a single B cell, or its progeny, secreting a single monoclonal antibody that generally binds to the desired antigen with a relatively high binding affinity, i.e. picomolar or better antigen binding affinities. By contrast, prior antibody selection methods tend to yield relatively few high affinity antibodies and therefore require extensive screening procedures to isolate an antibody with therapeutic potential. Without being bound by theory, it is hypothesized that the inventive protocol results in both in vivo B cell immunization of the host (primary immunization) followed by a second in vitro B cell immunization (secondary antigen priming step) that may enhance the ability and propensity of the recovered clonal B cells to secrete a single high affinity monoclonal antibody specific to the antigen target.
Third, it has been observed (as shown herein with IL-6 specific B cells) that the inventive B cell selection protocol reproducibly yields enriched B cells producing IgG's that are, on average, highly selective (antigen specific) to the desired target. In part based thereon, antigen-enriched B cells recovered by the inventive methods are believed to contain B cells capable of yielding the desired full complement of epitopic specificities as discussed above.
Fourth, it has been observed that the inventive B cell selection protocols, even when used with small antigens, i.e., peptides of 100 amino acids or less, e.g., 10-50 amino acids long, reproducibly give rise to a clonal B cell culture that secretes a single high affinity antibody to the small antigen, e.g., a peptide. This is highly surprising as it is generally quite difficult, labor intensive, and sometimes not even feasible to produce high affinity antibodies to small peptides. Accordingly, the invention can be used to produce therapeutic antibodies to desired peptide targets, e.g., viral, bacterial or autoantigen peptides, thereby allowing for the production of monoclonal antibodies with very discrete binding properties or even the production of a cocktail of monoclonal antibodies to different peptide targets, e.g., different viral strains. This advantage may especially be useful in the context of the production of a therapeutic or prophylactic vaccine having a desired valency, such as an HPV vaccine that induces protective immunity to different HPV strains.
Fifth, the inventive B cell selection protocol, particularly when used with B cells derived from rabbits, tends to reproducibly yield antigen-specific antibody sequences that are very similar to endogenous human immunoglobulins (around 90% similar at the amino acid level) and that contain CDRs that possess a length very analogous to human immunoglobulins and therefore require little or no sequence modification (typically at most only a few CDR residues may be modified in the parent antibody sequence and no framework exogenous residues introduced) in order to eliminate potential immunogenicity concerns. Thereby, the high antigen binding affinity of the recovered antibody sequences produced according to the inventive B cell and antibody selection protocol remains intact or substantially intact even with humanization.

In sum, the inventive method can be used to produce antibodies exhibiting higher binding affinities to more distinct epitopes by the use of a more efficient protocol than was previously known.

Described herein is a method for identifying a single B cell that secretes an antibody specific to a desired antigen and that optionally possesses at least one desired functional property such as affinity, avidity, cytolytic activity, and the like by a process including the following steps:
a. immunizing a host against an antigen;
b. harvesting B cells from the host;
c. enriching the harvested B cells to increase the frequency of antigen-specific cells;
d. creating at least one single cell suspension;
e. culturing a sub-population from the single cell suspension under conditions that favor the survival of a single antigen-specific B cell per culture well;
f. isolating less than 12 B cells from the sub-population; and
g. determining whether the single B cell produces an antibody specific to the antigen.

Typically, the inventive methods will further comprise an additional step of isolating and sequencing, in whole or in part, the polypeptide and nucleic acid sequences encoding the desired antibody. These sequences or modified versions or portions thereof can be expressed in desired host cells in order to produce recombinant antibodies to a desired antigen.

As noted previously, it is believed that the clonal population of B cells predominantly comprises antibody-secreting B cells producing antibody against the desired antigen. It is also believed based on experimental results obtained with several antigens and with different B cell populations that the clonally produced B cells and the isolated antigen-specific B cells derived therefrom produced according to the invention secrete a monoclonal antibody that is typically of relatively high affinity and moreover is capable of efficiently and reproducibly producing a selection of monoclonal antibodies of greater epitopic variability as compared to other methods of deriving monoclonal antibodies from cultured antigen-specific B cells. In an exemplary embodiment the population of immune cells used in such B cell selection methods will be derived from a rabbit. However, other hosts that produce antibodies, including non-human and human hosts, can alternatively be used as a source of immune B cells. It is believed that the use of rabbits as a source of B cells may enhance the diversity of monoclonal antibodies that may be derived by the inventive methods described herein. Also, the antibody sequences derived from rabbits according to the invention typically possess sequences having a high degree of sequence identity to human antibody sequences making them favored for use in humans since they should possess little antigenicity. In the course of humanization, the final humanized antibody contains a much lower foreign/host residue content, usually restricted to a subset of the host CDR residues that differ dramatically due to their nature versus the human target sequence used in the grafting. This enhances the probability of complete activity recovery in the humanized antibody protein.

The methods of antibody selection using an enrichment step disclosed herein include a step of obtaining a immune cell-containing cell population from an immunized host. Methods of obtaining an immune cell-containing cell population from an immunized host are known in the art and generally include inducing an immune response in a host and harvesting cells from the host to obtain one or more cell populations. The response can be elicited by immunizing the host against a desired antigen. Alternatively, the host used as a source of such immune cells can be naturally exposed to the desired antigen such as an individual who has been infected with a particular pathogen such as a bacterium or virus or alternatively has mounted a specific antibody response to a cancer that the individual is afflicted with.

Host animals are well-known in the art and include, but are not limited to, human, rabbit, mouse, rat, chicken cow, pig, guinea pig, goat, and sheep. When exposed to an antigen, the host produces antibodies as part of the native immune response to the antigen. As mentioned, the immune response can occur naturally, as a result of disease, or it can be induced by immunization with the antigen. Immunization can be performed by any method known in the art, such as, by one or more injections of the antigen with or without an agent to enhance immune response, such as complete or incomplete Freund's adjuvant. As an alternative to immunizing a host animal *in vivo*, the method can comprise immunizing a host cell culture *in vitro.*

After allowing time for the immune response (e.g., as measured by serum antibody detection), host animal cells are harvested to obtain one or more cell populations. In a preferred embodiment, a harvested cell population is screened for antibody binding strength and/or antibody functionality. A harvested cell population is preferably from at least one of the spleen, lymph nodes, bone marrow, and/or peripheral blood mononuclear cells (PBMCs). The cells can be harvested from more than one source and pooled. Certain sources may be preferred for certain antigens. For example, the spleen, lymph nodes, and PBMCs are preferred for IL-6; the lymph nodes are preferred for TNF; and the spleen and lymph nodes are preferred for Aβ. The cell population is harvested about 20 to about 90 days or increments therein after immunization, preferably about 50 to about 60 days. A harvested cell population and/or a single cell suspension therefrom can be enriched, screened, and/or cultured for antibody selection. The frequency of antigen-specific cells within a harvested cell population is usually about 1% to about 5%, or increments therein.

In one embodiment, a single cell suspension from a harvested cell population is enriched, preferably by using Miltenyi beads. From the harvested cell population having a frequency of antigen-specific cells of about 1% to about 5%, an enriched cell population is thus derived having a frequency of antigen-specific cells approaching 100%.

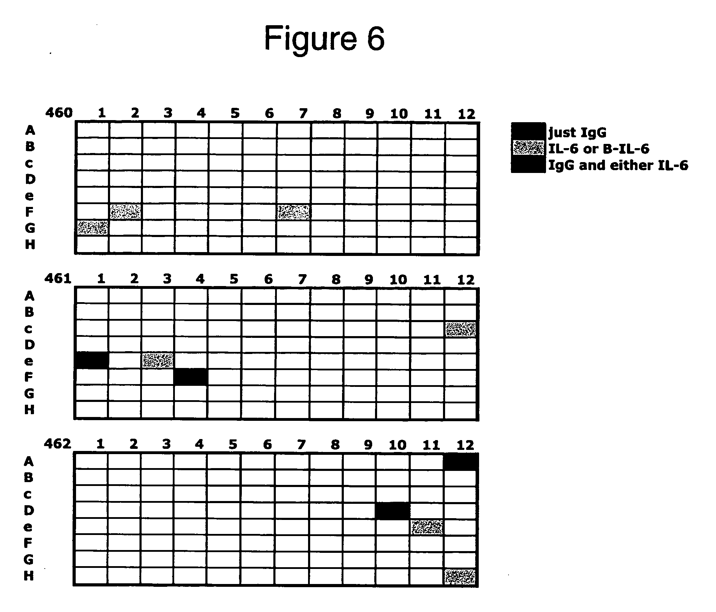

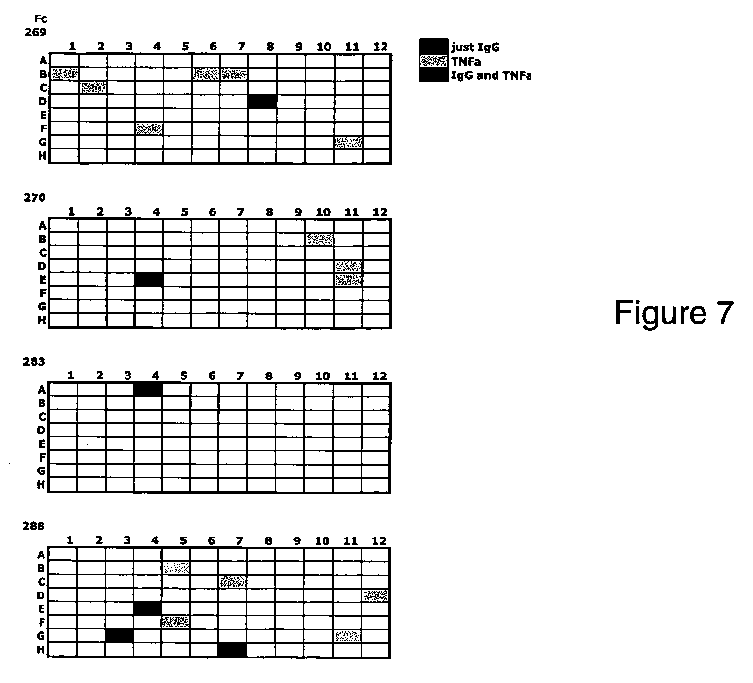

The method of antibody selection using an enrichment step includes a step of producing antibodies from at least one antigen-specific cell from an enriched cell population. Methods of producing antibodies *in vitro* are well known in the art, and any suitable method can be employed. In one embodiment, an enriched cell population, such as an antigen-specific single cell suspension from a harvested cell population, is plated at various cell densities, such as 50, 100, 250, 500, or other increments between 1 and 1000 cells per well. Preferably, the sub-population comprises no more than about 10,000 antigen-specific, antibody-secreting cells, more preferably about 50-10,000, about 50-5,000, about 50-1,000, about 50-500, about 50-250 antigen-specific, antibody-secreting cells, or increments therein. Then, these sub-populations are cultured with suitable medium on a feeder layer, preferably under conditions that favor the survival of a single proliferating antibody-secreting cell per culture well. The feeder layer, generally comprised of irradiated cell matter, does not constitute part of the cell population. The cells are cultured in a suitable media for a time sufficient for antibody production, for example about 1 day to about 2 weeks, about 1 day to about 10 days, at least about 3 days, about 3 to about 5 days, about 5 days to about 7 days, or other increments therein. Preferably, a single antibody-producing cell and progeny thereof survives in each well, thereby providing a clonal population of antigen-specific B cells in each well. At this stage, the immunoglobulin G (IgG) produced by the clonal population is highly correlative with antigen specificity. In a preferred embodiment, the IgGs exhibit a correlation with antigen specificity that is greater than about 50%, more preferably greater than 70%, 85%, 90%, 95%, 99%, or increments therein. See Fig. 6 and Fig. 7, which demonstrate exemplary correlations for IL-6 and huTNF-α, respectively. The correlations were demonstrated by setting up B cell cultures under limiting conditions to establish single antigen-specific antibody products per well. Antigen-specific versus general IgG synthesis was compared. Three populations were observed: IgG that recognized a single formate of antigen (biotinylated and direct coating), detectable IgG and antigen recognition irrespective of immobilization, and IgG production alone. IgG production was highly correlated with antigen-specificity.

A supernatant containing the antibodies is optionally collected, which can be can be enriched, screened, and/or cultured for antibody selection according to the steps described above. In one embodiment, the supernatant is enriched (preferably by an antigen-specificity assay, especially an ELISA assay) and/or screened for antibody functionality.

In another embodiment, the enriched, preferably clonal, antigen-specific B cell population from which a supernatant described above is optionally screened in order to detect the presence of the desired secreted monoclonal antibody is used for the isolation of a few B cells, preferably a single B cell, which is then tested in an appropriate assay in order to confirm the presence of a single antibody-producing B cell in the clonal B cell population. In one embodiment about 1 to about 20 cells are isolated from the clonal B cell population, preferably less than about 15, 12, 10, 5, or 3 cells, or increments therein, most preferably a single cell. The screen is preferably effected by an antigen-specificity assay, especially a halo assay. The halo assay can be performed with the full length protein, or a fragment thereof. The antibody-containing supernatant can also be screened for at least one of: antigen binding affinity; agonism or antagonism of antigen-ligand binding, induction or inhibition of the proliferation of a specific target cell type; induction or inhibition of lysis of a target cell, and induction or inhibition of a biological pathway involving the antigen.

The identified antigen-specific cell can be used to derive the corresponding nucleic acid sequences encoding the desired monoclonal antibody. (An AluI digest can confirm that only a single monoclonal antibody type is produced per well.) As mentioned above, these sequences can be mutated, such as by humanization, in order to render them suitable for use in human medicaments.

As mentioned, the enriched B cell population used in the inventive process can also be further enriched, screened, and/or cultured for antibody selection according to the steps described above which can be repeated or performed in a different order. In a preferred embodiment, at least one cell of an enriched, preferably clonal, antigen-specific cell population is isolated, cultured, and used for antibody selection.

Described herein is a method comprising:
a. harvesting a cell population from an immunized host to obtain a harvested cell population;
b. creating at least one single cell suspension from a harvested cell population;
c. enriching at least one single cell suspension, preferably by chromatography, to form a first enriched cell population;
d. enriching the first enriched cell population, preferably by ELISA assay, to form a second enriched cell population which preferably is clonal, i.e., it contains only a single type of antigen-specific B cell;
e. enriching the second enriched cell population, preferably by halo assay, to form a third enriched cell population containing a single or a few number of B cells that produce an antibody specific to a desired antigen; and
f. selecting an antibody produced by an antigen-specific cell isolated from the third enriched cell population.

The method can further include one or more steps of screening the harvested cell population for antibody binding strength (affinity, avidity) and/or antibody functionality. Suitable screening steps include, but are not limited to, assay methods that detect: whether the antibody produced by the identified antigen-specific B cell produces an antibody possessing a minimal antigen binding affinity, whether the antibody agonizes or antagonizes the binding of a desired antigen to a ligand; whether the antibody induces or inhibits the proliferation of a specific cell type; whether the antibody induces or elicits a cytolytic reaction against target cells; whether the antibody binds to a specific epitope; and whether the antibody modulates (inhibits or agonizes) a specific biological pathway or pathways involving the antigen.

Similarly, the method can include one or more steps of screening the second enriched cell population for antibody binding strength and/or antibody functionality.

The method can further include a step of sequencing the polypeptide sequence or the corresponding nucleic acid sequence of the selected antibody. The method can also include a step of producing a recombinant antibody using the sequence, a fragment thereof, or a genetically modified version of the selected antibody. Methods for mutating antibody sequences in order to retain desired properties are well known to those skilled in the art and include humanization, chimerisation, production of single chain antibodies; these mutation methods can yield recombinant antibodies possessing desired effector function, immunogenicity, stability, removal or addition of glycosylation, and the like. The recombinant antibody can be produced by any suitable recombinant cell, including, but not limited to mammalian cells such as CHO, COS, BHK, HEK-293, bacterial cells, yeast cells, insect cells, and amphibian cells. In one embodiment, the antibodies are expressed in polyploidal yeast cells, i.e., diploid yeast cells, particularly *Pichia.*

Described herein is a method comprises:
a. immunizing a host against an antigen to yield host antibodies;
b. screening the host antibodies for antigen specificity and neutralization;
c. harvesting B cells from the host;
d. enriching the harvested B cells to create an enriched cell population having an increased frequency of antigen-specific cells;
e. culturing one or more sub-populations from the enriched cell population under conditions that favor the survival of a single B cell to produce a clonal population in at least one culture well;
f. determining whether the clonal population produces an antibody specific to the antigen;
g. isolating a single B cell; and
h. sequencing the nucleic acid sequence of the antibody produced by the single B cell.

To further articulate the invention described above, we provide the following nonlimiting examples.

### EXAMPLES

### Example 1: Production of Enriched Antigen-Specific B Cell Antibody Culture

Panels of antibodies are derived by immunizing traditional antibody host animals to exploit the native immune response to a target antigen of interest. Typically, the host used for immunization is a rabbit or other host that produces antibodies using a similar maturation process and provides for a population of antigen-specific B cells producing antibodies of comparable diversity, e.g., epitopic diversity. The initial antigen immunization can be conducted using complete Freund's adjuvant (CFA), and the subsequent boosts effected with incomplete adjuvant. At about 50-60 days after immunization, preferably at day 55, antibody titers are tested, and the Antibody Selection (ABS) process is initiated if appropriate titers are established. The two key criteria for ABS initiation are potent antigen recognition and function-modifying activity in the polyclonal sera.

At the time positive antibody titers are established, animals are sacrificed and B cell sources isolated. These sources include: the spleen, lymph nodes, bone marrow, and peripheral blood mononuclear cells (PBMCs). Single cell suspensions are generated, and the cell suspensions are washed to make them compatible for low temperature long term storage. The cells are then typically frozen.

To initiate the antibody identification process, a small fraction of the frozen cell suspensions are thawed, washed, and placed in tissue culture media. These suspensions are then mixed with a biotinylated form of the antigen that was used to generate the animal immune response, and antigen-specific cells are recovered using the Miltenyi magnetic bead cell selection methodology. Specific enrichment is conducted using streptavidin beads. The enriched population is recovered and progressed in the next phase of specific B cell isolation.

### Example 2: Production of Clonal, Antigen-Specific B Cell-Containing Culture

Enriched B cells produced according to Example 1 are then plated at varying cell densities per well in a 96 well microtiter plate. Generally, this is at 50, 100, 250, or 500 cells per well with 10 plates per group. The media is supplemented with 4% activated rabbit T cell conditioned media along with 50K frozen irradiated EL4B feeder cells. These cultures are left undisturbed for 5-7 days at which time supernatant-containing secreted antibody is collected and evaluated for target properties in a separate assay setting. The remaining supernatant is left intact, and the plate is frozen at -70°C. Under these conditions, the culture process typically results in wells containing a mixed cell population that comprises a clonal population of antigen-specific B cells, i.e., a single well will only contain a single monoclonal antibody specific to the desired antigen.

### Example 3: Screening of Antibody Supernatants for Monoclonal Antibody of Desired Specificity and/or Functional Properties

Antibody-containing supernatants derived from the well containing a clonal antigen-specific B cell population produced according to Example 2 are initially screened for antigen recognition using ELISA methods. This includes selective antigen immobilization (e.g., biotinylated antigen capture by streptavidin coated plate), non-specific antigen plate coating, or alternatively, through an antigen build-up strategy (e.g., selective antigen capture followed by binding partner addition to generate a heteromeric protein-antigen complex). Antigen-positive well supernatants are then optionally tested in a function-modifying assay that is strictly dependant on the ligand. One such example is an *in vitro* protein-protein interaction assay that recreates the natural interaction of the antigen ligand with recombinant receptor protein. Alternatively, a cell-based response that is ligand dependent and easily monitored (e.g., proliferation response) is utilized. Supernatant that displays significant antigen recognition and potency is deemed a positive well. Cells derived from the original positive well are then transitioned to the antibody recovery phase.

### Example 4: Recovery of Single, Antibody-Producing B Cell of Desired Antigen Specificity

A few number of cells are isolated from a well that contains a clonal population of antigen-specific B cells (produced according to Example 2 or 3), which secrete a single antibody sequence. The isolated cells are then assayed to isolate a single, antibody-secreting cell. Dynal streptavidin beads are coated with biotinylated target antigen under buffered medium to prepare antigen-containing microbeads compatible with cell viability. Next antigen-loaded beads, antibody-producing cells from the positive well, and a fluorescein isothiocyanate (FITC)-labeled anti-host H&L IgG antibody (as noted, the host can be any mammalian host, e.g., rabbit, mouse, rat, etc.) are incubated together at 37°C. This mixture is then re-pipetted in aliquots onto a glass slide such that each aliquot has on average a single, antibody-producing B-cell. The antigen-specific, antibody-secreting cells are then detected through fluorescence microscopy. Secreted antibody is locally concentrated onto the adjacent beads due to the bound antigen and provides localization information based on the strong fluorescent signal. Antibody-secreting cells are identified via FITC detection of antibody-antigen complexes formed adjacent to the secreting cell. The single cell found in the center of this complex is then recovered using a micromanipulator. The cell is snap-frozen in an eppendorf PCR tube for storage at -80°C until antibody sequence recovery is initiated.

### Example 5: Isolation of Antibody Sequences From Antigen-Specific B Cell

Antibody sequences are recovered using a combined RT-PCR based method from a single isolated B-cell produced according to Example 4 or an antigenic specific B cell isolated from the clonal B cell population obtained according to Example 2. Primers are designed to anneal in conserved and constant regions of the target immunoglobulin genes (heavy and light), such as rabbit immunoglobulin sequences, and a two-step nested PCR recovery step is used to obtain the antibody sequence. Amplicons from each well are analyzed for recovery and size integrity. The resulting fragments are then digested with AluI to fingerprint the sequence clonality. Identical sequences display a common fragmentation pattern in their electrophoretic analysis. Significantly, this common fragmentation pattern which proves cell clonality is generally observed even in the wells originally plated up to 1000 cells/well. The original heavy and light chain amplicon fragments are then restriction enzyme digested with HindIII and XhoI or HindIII and BsiwI to prepare the respective pieces of DNA for cloning. The resulting digestions are then ligated into an expression vector and transformed into bacteria for plasmid propagation and production. Colonies are selected for sequence characterization.

### Example 6: Recombinant Production of Monoclonal Antibody of Desired Antigen Specificity and/or Functional Properties

Correct full-length antibody sequences for each well containing a single monoclonal antibody is established and miniprep DNA is prepared using Qiagen solid-phase methodology. This DNA is then used to transfect mammalian cells to produce recombinant full-length antibody. Crude antibody product is tested for antigen recognition and functional properties to confirm the original characteristics are found in the recombinant antibody protein. Where appropriate, large-scale transient mammalian transfections are completed, and antibody is purified through Protein A affinity chromatography. K_{d} is assessed using standard methods (e.g., Biacore) as well as IC₅₀ in a potency assay.

### Example 7: Preparation of Antibodies that Bind Human IL-6

By using the antibody selection protocol described herein, one can generate an extensive panel of antibodies. The antibodies have high affinity towards IL-6 (single to double digit pM K_{d}) and demonstrate potent antagonism of IL-6 in multiple cell-based screening systems (T1165 and HepG2). Furthermore, the collection of antibodies display distinct modes of antagonism toward IL-6-driven processes.

### Immunization Strategy

Rabbits were immunized with huIL-6 (R&R). Immunization consisted of a first subcutaneous (sc) injection of 100 µg in complete Freund's adjuvant (CFA) (Sigma) followed by two boosts, two weeks apart, of 50 µg each in incomplete Freund's adjuvant (IFA) (Sigma). Animals were bled on day 55, and serum titers were determined by ELISA (antigen recognition) and by non-radioactive proliferation assay (Promega) using the T1165 cell line.

### Antibody Selection Titer Assessment

Antigen recognition was determined by coating Immulon 4 plates (Thermo) with 1 µg/ml of huIL-6 (50 µl/well) in phosphate buffered saline (PBS, Hyclone) overnight at 4°C. On the day of the assay, plates were washed 3 times with PBS /Tween 20 (PBST tablets, Calbiochem). Plates were then blocked with 200 µl/well of 0.5% fish skin gelatin (FSG, Sigma) in PBS for 30 minutes at 37°C. Blocking solution was removed, and plates were blotted. Serum samples were made (bleeds and pre-bleeds) at a starting dilution of 1:100 (all dilutions were made in FSG 50 µl/well) followed by 1:10 dilutions across the plate (column 12 was left blank for background control). Plates were incubated for 30 minutes at 37°C. Plates were washed 3 times with PBS/Tween 20. Goat anti-rabbit FC-HRP (Pierce) diluted 1:5000 was added to all wells (50 µl/well), and plates were incubated for 30 minutes at 37°C. Plates were washed as described above. 50 µl/well of TMB-Stable stop (Fitzgerald Industries) was added to plates, and color was allowed to develop, generally for 3 to 5 minutes. The development reaction was stopped with 50 µl/well 0.5 M HCl. Plates were read at 450 nm. Optical density (OD) versus dilution was plotted using Graph Pad Prizm software, and titers were determined.

### Functional Titer Assessment

The functional activity of the samples was determined by a T1165 proliferation assay. T1165 cells were routinely maintained in modified RPMI medium (Hyclone) supplemented with Hepes, sodium pyruvate, sodium bicarbonate, L-glutamine, high glucose, penicillin/streptomycin, 10% heat inactivated FBS (all supplements from Hyclone), 2-mercaptoethanol (Sigma), and 10 ng/ml of huIL-6 (R&D). On the day of the assay, cell viability was determined by trypan blue (Invitrogen), and cells were seeded at a fixed density of 20,000 cells/well. Prior to seeding, cells were washed twice in medium described above without human-IL-6 (by centrifuging at 13000 rpm/ 5 minutes and discarding the supernatant). After the last wash, cells were resuspended in the same medium used for washing in a volume equivalent to 50 µl/well. Cells were set aside at room temperature.

In a round-bottom, 96-well plate (Costar), serum samples were added starting at 1:100, followed by a 1:10 dilution across the plate (columns 2 to 10) at 30 µl/well in replicates of 5 (rows B to F: dilution made in the medium described above with no huIL-6). Column 11 was medium only for IL-6 control. 30 µl/well of huIL-6 at 4x concentration of the final EC₅₀ (concentration previously determined) were added to all wells (huIL-6 was diluted in the medium described above). Wells were incubated for 1 hour at 37°C to allow antibody binding to occur. After 1 hour, 50 µl/well of antibody-antigen (Ab-Ag) complex were transferred to a flat-bottom, 96-well plate (Costar) following the plate map format laid out in the round-bottom plate. On Row G, 50 µl/well of medium were added to all wells (columns 2 to 11) for background control. 50 µl/well of the cell suspension set aside were added to all wells (columns 2 to 11, rows B to G). On Columns 1 and 12 and rows A and H, 200 µl/well of medium was added to prevent evaporation of test wells and to minimize edge effect. Plates were incubated for 72 h at 37°C in 4% CO₂. At 72 h, 20 µl/well of CellTiter96 (Promega) reagents was added to all test wells per manufacturer protocol, and plates were incubated for 2 h at 37°C. At 2 h, plates were gently mixed on an orbital shaker to disperse cells and to allow homogeneity in the test wells. Plates were read at 490 nm wavelength. Optical density (OD) versus dilution was plotted using Graph Pad Prizm software, and functional titer was determined. A positive assay control plate was conducted as described above using MAB2061 (R&D Systems) at a starting concentration of 1 µg/ml (final concentration) followed by 1:3 dilutions across the plate.

### Tissue Harvesting:

Once acceptable titers were established, the rabbit(s) were sacrificed. Spleen, lymph nodes, and whole blood were harvested (R&R) and processed as follows:
Spleen and lymph nodes were processed into a single cell suspension by disassociating the tissue and pushing through sterile wire mesh at 70 µm (Fisher) with a plunger of a 20 cc syringe. Cells were collected in the modified RPMI medium described above without huIL-6, but with low glucose. Cells were washed twice by centrifugation. After the last wash, cell density was determined by trypan blue. Cells were centrifuged at 1500 rpm for 10 minutes; the supernatant was discarded. Cells were resuspended in the appropriate volume of 10% dimethyl sulfoxide (DMSO, Sigma) in FBS (Hyclone) and dispensed at 1 ml/vial. Vials were then stored at -70°C for 24 h prior to being placed in a liquid nitrogen (LN₂) tank for long-term storage.

Peripheral blood mononuclear cells (PBMCs) were isolated by mixing whole blood with equal parts of the low glucose medium described above without FBS. 35 ml of the whole blood mixture was carefully layered onto 8 ml of Lympholyte Rabbit (Cedarlane) into a 45 ml conical tube (Corning) and centrifuged 30 minutes at 2500 rpm at room temperature without brakes. After centrifugation, the PBMC layers were carefully removed using a glass Pasteur pipette (VWR), combined, and placed into a clean 50 ml vial. Cells were washed twice with the modified medium described above by centrifugation at 1500 rpm for 10 minutes at room temperature, and cell density was determined by trypan blue staining. After the last wash, cells were resuspended in appropriate volume of 10% DMSO/FBS medium and frozen as described above.

### B cell culture

On the day of setting up B cell culture, PBMC, splenocyte, or lymph node vials were thawed for use. Vials were removed from LN₂ tank and placed in a 37°C water bath until thawed. Contents of vials were transferred into 15 ml conical centrifuge tube (Corning) and 10 ml of modified RPMI described above was slowly added to the tube. Cells were centrifuged for 5 minutes at 1.5K rpm, and the supernatant was discarded. Cells were resuspended in 10 ml of fresh media. Cell density and viability was determined by trypan blue. Cells were washed again and resuspended at 1E07 cells/80 µl medium. Biotinylated huIL-6 was added to the cell suspension at a final concentration of 3 µg/ml and incubated for 30 minutes at 4°C. Unbound B-huIL-6 was removed with two 10 ml washes of phosphate-buffered fluoride (PBF):Ca/Mg free PBS (Hyclone), 2 mM ethylenediamine tetraacetic acid (EDTA), 0.5% bovine serum albumin (BSA) (Sigma-biotin free). After the second wash, cells were resuspended at 1E07 cells/80 µl PBF. 20 µl of MACS® streptavidin beads (Milteni)/10E7 cells were added to the cell suspension. Cells were incubated at 4°C for 15 minutes. Cells were washed once with 2 ml of PBF/10E7 cells. After washing, the cells were resuspended at 1E08 cells/500 µl of PBF and set aside. A MACS® MS column (Milteni) was pre-rinsed with 500 ml of PBF on a magnetic stand (Milteni). Cell suspension was applied to the column through a pre-filter, and unbound fraction was collected. The column was washed with 1.5 ml of PBF buffer. The column was removed from the magnet stand and placed onto a clean, sterile 5 ml Polypropylene Falcon tube. 1 ml of PBF buffer was added to the top of the column, and positive selected cells were collected. The yield and viability of positive and negative cell fraction was determined by trypan blue staining. Positive selection yielded an average of 1% of the starting cell concentration.

A pilot cell screen was established to provide information on seeding levels for the culture. Three 10-plate groups (a total of 30 plates) were seeded at 50, 100, and 200 enriched B cells/well. In addition, each well contained 50K cell/well of irradiated EL-4.B5 cells (5,000 Rads) and an appropriate level of T cell supernatant (ranging from 1-5% depending on preparation) in high glucose modified RPMI medium at a final volume of 250 µl/well. Cultures were incubated for 5 to 7 days at 37°C in 4% CO₂.

### Identification of Selective Antibody Secreting B Cells

Cultures were tested for antigen recognition and functional activity between days 5 and 7.

### Antigen Recognition Screening

The ELISA format used is as described above except 50 µl of supernatant from the B cell cultures (BCC) wells (all 30 plates) was used as the source of the antibody. The conditioned medium was transferred to antigen-coated plates. After positive wells were identified, the supernatant was removed and transferred to a 96-well master plate(s). The original culture plates were then frozen by removing all the supernatant except 40 µl/well and adding 60 µl/well of 16% DMSO in FBS. Plates were wrapped in paper towels to slow freezing and placed at -70°C.

### Functional Activity Screening

Master plates were then screened for functional activity in the T1165 proliferation assay as described before, except row B was media only for background control, row C was media + IL-6 for positive proliferation control, and rows D-G and columns 2-11 were the wells from the BCC (50 µl/well, single points). 40 µl of IL-6 was added to all wells except the media row at 2.5 times the EC₅₀ concentration determined for the assay. After 1 h incubation, the Ab/Ag complex was transferred to a tissue culture (TC) treated, 96-well, flat-bottom plate. 20 µl of cell suspension in modified RPMI medium without huIL-6 (T1165 at 20,000 cells/well) was added to all wells (100 µl final volume per well). Background was subtracted, and observed OD values were transformed into % of inhibition.

### B cell recovery

Plates containing wells of interest were removed from -70°C, and the cells from each well were recovered with 5-200 µl washes of medium/well. The washes were pooled in a 1.5 ml sterile centrifuge tube, and cells were pelleted for 2 minutes at 1500 rpm.

The tube was inverted, the spin repeated, and the supernatant carefully removed. Cells were resuspended in 100 µl/tube of medium. 100 µl biotinylated IL-6 coated streptavidin M280 dynabeads (Invitrogen) and 16 µl of goat anti-rabbit H&L IgG-FITC diluted 1:100 in medium was added to the cell suspension.

20 µl of cell/beads/FITC suspension was removed, and 5 µl droplets were prepared on a glass slide (Corning) previously treated with sigmacote (Sigma) and an impermeable barrier (35 to 40 droplets/slide). Parafin oil (JT Baker) was added to submerge the droplets, and the slide was incubated for 90 minutes at 37°C, 4% CO₂ in the dark.

Specific B cells that produce antibody can be identified by the fluorescent ring around them due to antibody secretion, recognition of the bead-associated biotinylated antigen, and subsequent detection by the fluorescent-IgG detection reagent. Once a cell of interest was identified, the cell in the center of the fluorescent ring was recovered via a micromanipulator (Eppendorf). The single cell synthesizing and exporting the antibody was transferred into a 250 µl microcentrifuge tube and placed in dry ice. After recovering all cells of interest, these were transferred to -70°C for long-term storage.

### Example 8: Preparation of Antibodies that Bind HuTNF-α

By using the antibody selection protocol described herein, one can generate a collection of antibodies that exhibit potent functional antagonism of TNF-α. The antibodies elucidate a variety of TNF-α epitopes and thus may provide useful alternatives to, or adjunctives with, antibodies that target previously identified TNF-α epitopes, such as Remicade® (infliximab).

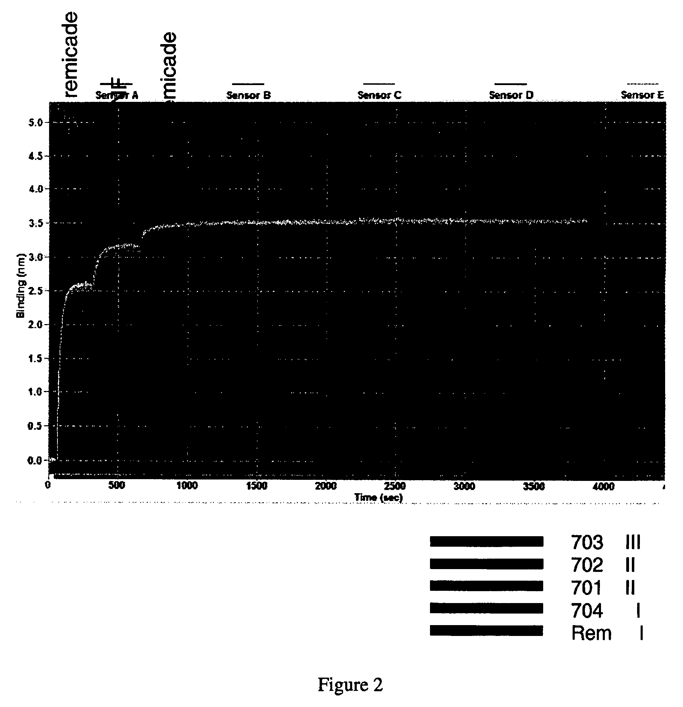

A screening method can be employed to identify antibodies that bind alternative TNF-α epitopes, while retaining significant functional antagonism. After the primary antigen-recognition screen, positive BCC wells were tested for functional antagonism towards TNF-α as well as for epitope competition, e.g., competition with infliximab. Unique epitope recognition was established by ForteBio Octet antibody-TNF-α binding competition studies. See Fig. 2. BCC wells that displayed functional activity as well as lack of competition were pursued, and the coding sequences for the antibody present in these wells recovered. The majority of the recovered sequences displayed the original target characteristics: potent antigen recognition, functional antagonism, and distinct epitope recognition. Thus, the resulting antibody collection established multiple novel epitope regions associated with potent functional antagonism.

### Immunization Strategy:

Rabbits were immunized with TNF-α (R&D # 210-TA) using an identical protocol as that described for huIL-6.

### ABS Titer Assessment

Antigen recognition assay was determined for TNF-α by the protocol described for huIL-6, except plates were coated with this cytokine at the concentration described above.

### Functional Titer Assessment

The functional activities of the samples were determined by a TNF-α stimulated L929 and/or WEHI cytotoxic assay. L929 or WEHI cells were routinely maintained in the medium described above without huIL-6. On the day of the assay, cell density was determined by trypan blue. Cells were resuspended at 1E06 cells/ml and plated at 50 µl/well (volume was adjusted to number of samples and replicates) in sterile flat-bottom 96-well tissue culture plates. Plates were incubated for 2 h at 37°C.

Separately, in a round-bottom 96-well plate, serum samples were added at a 1:100 dilution (in the described media) followed by 1:10 dilution across the plate (columns 2-10, column 11 was media only for TNF-α control), 50 µl/well in replicates of 5 (rows B-F, row G was media only for background control). 50 µl/well of media containing TNF-α at a concentration 4 times the final EC₅₀ (concentration was previously determined for each lot) and 1 µg/ml of Actinomycin D was added to all sample wells except row F. Plates were incubated for 1 h at 37°C.

At 1 h, 50 µl of the Serum/Ag complex and controls were transferred to the 96-well flat-bottom plates containing 50 µl/well of responder cells at a fixed density (final volume= 100 µl/well) and incubated for 24 h at 37°C. (Columns 1 and 12 and rows A and H were filled with 200 µl of media to prevent evaporation and cause edge effect.)

At 24 h, 20 µl/well of CellTiter96 reagent (Promega) was added to all test wells per the manufacturer protocol, and plates were incubated for 2 h at 37°C. After 2 h, plates were gently shaken to allow homogeneity in the test wells. Plates were read at 490 nm wavelength. OD versus dilution were plotted using Graph Pad Prizm (non-linear sigmoid dose/response curve was used), and functional titer was determined.

### Tissue Harvesting

Rabbit spleen, lymph nodes, and whole blood were harvested, processed, and frozen as described above for huIL-6.

### B Cell Culture (BCC)

B cell cultures were prepared as described for huIL-6, except cell enrichment was done using biotinylated huTNF-α.

### Antigen Recognition Screening

Antigen recognition screening was performed as described above as single points.

### Functional Activity Screening

Functional activity screening was performed by a WEHI cytotoxic assay. Supernatant from master plate(s) was tested in the TNF-α stimulated WEHI cytotoxic assay (as described above) as single points. Supernatants were tested as neat according to the following template:
Row F is media only for background control (50 µl/well).
Row G is media + TNF-α for positive cytotoxic control.
Rows B-E and columns 2-11 are the wells from the BCC (40 µl/well, single points).

40 µl of TNF-α + Actinomycin D was added to all wells (except the media row) at 4 times the EC₅₀ concentration determined for the assay. After 1 h incubation, the Ab/Ag complex was transferred to a TC-treated 96-well flat-bottom plate. 20 µl of cell suspension (WEHI at 1E06 cells/ml) was added to all wells (final volume: 100 µl/well), and the plates were incubated for 24 h at 37°C. At 24 h, CellTiter96 reagent was added per manufacturer instructions. Plates were read at 490 nm wavelength, background was subtracted from wells, and OD values were transformed into % inhibition.

### Secondary Functional Activity Assay for Recombinant Antibodies: Blocking of IL-6 Expression by HUVEC cells treated with huTNF-α

Human umbilical vein endothelial cells (HUVECs) were routinely maintained in endothelial growth medium (EGM) medium and appropriated HUVEC supplements (Cambrex). On the day of the assay, HUVEC viability was determined by trypan blue. The cells were resuspended at 5E05/ml in the appropriate volume of medium necessary for the assay (100 µl/well). Cells were plated in middle wells of 96-well flat-bottom culture plates, and 200 µl medium was added to all outside wells to prevent evaporation. The plate was incubated for 24 h at 37°C.

At 24 h, the appropriate antibody dilutions are made in EGM at 4x the desired final concentration. (Starting antibody concentration was 1 ug/ml; a 1:3 dilution was performed across the plate, except for last row.) The same volume of rhuTNF-α in EGM (4x the desired final concentration) was added to the wells. The plate was incubated for 1 h at 37°C to form the antibody/antigen complex. At 1 h, 50 µl of media from the HUVEC culture plate was removed and discarded. 50 µl Ab-Ag mixture was added, and the plate was incubated for 48 h at 37°C. Standard positive and negative controls were included: huTNF-α only (column 11), medium only (No Ab/ No TNF) for background growth (row G).

At 48 h, conditioned medium IL-6 levels were assessed by ELISA. An Immulon plate was coated with 1 µg/ml goat anti-huIL-6 at 50 µl/well, overnight at 4°C, or room temperature for 1 hour. The plate was washed in PBS + 0.5% Tween 20 in a plate washer (200 µl/well; 3 times). The plate was blocked with 200 µl/well FSG for 1 hour at room temperature. The blocking solution was aspirated, and the plate was blotted. The huIL-6 standard was set on rows A and B (duplicates), starting at 1 µg/ml and diluted 1:3 across the plate (all dilutions made in FSG) leaving column 12 as blank. Samples from HUVEC culture were added to the wells below standard curve and incubated for 1 hour at room temperature. Wash was repeated. 1 µg/ml ALD515v5 (anti-huIL-6) was added at 50 µl/well to the plate and incubated for 1 hour at room temperature. Wash was repeated. Secondary anti-human IgG Fc HRP at 1:5000 dilution was added at 50 µl/well and incubated for 45 minutes at room temperature. Wash was repeated. Assay was developed with 50 µl/well 3,3',5,5' tetramethylbenzidine (TMB) for a minimum of 5 minutes. The reaction was stopped with 50 µl/well HCl**,** and the plate was read at 450 nm in a plate reader. Data was analyzed using Graph Pad Prizm.

### B Cell Recovery

The foci protocol was performed as described for huIL-6, except using B-huTNF-α.

### Example 9: Preparation of Antibodies that Bind Aβ

Two forms of Aβ were used to generate immune responses in rabbits: Aβ 1-40 and Aβ 1-42 peptides. By using the antibody selection protocol described herein, one can identify antibodies that selectively recognize each form of Aβ by C-terminal amino acid sequences unique epitope context. In addition, one can identify antibodies with potent antigen recognition in the central region of the peptide as well as the N-terminus. The screening strategy was as follows: (1) conduct the primary BCC screening using the antigen specific to the immunization protocol, and (2) perform selectivity screens using smaller peptides representing the regions of interest (N-terminal (1-12), central (10-35), and C-terminal (34-40 and 36-42), respectively). The coding sequences of the antibodies generated by the BCCs in wells identified with desired secondary screen selectivity were recovered by using small peptide species in the recovery phase. The novel use of small peptides in this process proved highly efficient; potent selective antibody recovery was established supporting the use of small peptides (6-mers or greater) in the foci recovery process, especially in cases where restricted epitopes in the target antigen are desired.

### Immunization Strategy

Two rabbits for each Aβ peptide were immunized with the following peptides obtained from Anaspec: Aβ 1-40, Aβ 1-42, KLH-Cys-Aβ 1-40, and Aβ 1-42-KLH from American Peptide. Immunization was performed according to the procedure described for huIL-6.

### ABS Titer Assessment

Antigen recognition assay was determined by the ELISA protocol described for huIL-6, except streptavidin coated plates (Sigma) were employed to immobilize the antigen using biotinylated peptides Aβ biotin-1-40 and Aβ biotin-1-42 at the concentration mentioned before in 1x PBS (Hyclone) for 1 h at room temperature. The subsequent steps are described above. Commercially available antibodies to the specific peptides were used as controls. Serum samples were screened against all peptides to determine titer specificity.

### Tissue harvesting:

Rabbit spleen, lymph nodes, and whole blood was harvested, processed, and frozen as described above for huIL-6.

### B Cell Culture

B cell culture was set as described for huIL-6, except for B cell enrichment, the following reagents and strategy were used. For animals immunized with the Aβ 1-40 peptide, Aβ b-1-40 was used for enrichment. For animals immunized with the Aβ 1-42 peptide, a first pass enrichment was done using Aβ b-1-42, and the flow through was further enriched using Aβ b-1-40. Plates were labeled accordingly to the enrichment procedure.

### Antigen Recognition Screening

Plates were screened by the ELISA protocol described above, using the biotinylated peptides. Plates were primarily screened against both Aβ 1-40 and Aβ 1-42 peptides. To further delineate the epitope location and to identify antibodies specific to the N-terminus, central region, and the C-terminus of each peptide, peptide fragments representing each of these regions were used in secondary screens. Once positive wells were identified, they were combined into a master plate, and further peptide mapping was performed by an ELISA subtraction protocol.

Wells of streptavidin coated plates were coated in 1x PBS, 50 µl/well, 1 µg/ml with each of the following peptides (number of wells coated depended on number of positive wells identified on primary screening): Aβ b-10-35, Aβ b-34-40, Aβ b-36-44, Aβ b-38-42 (all prepared by custom synthesis; American peptide). 50 µl/ well of supernatant from positive wells were serially incubated (1 h at room temperature) with each of the peptides coated onto the well (wells with no supernatant were filled with FSG at each of the serial incubation). The remaining ELISA protocol was performed as described previously. Wells with the desired antibody characteristics were selected for B cell recovery.

### B Cell Recovery

Selected wells were focied as described above, except the biotinylated peptide reagent for each region was employed with the appropriate positive specific for the region mapped by the ELISA subtraction protocol.

### Example 10: Recovery of Isolated B Cell Variable Light and Heavy Chain Sequence and

### Expression of Recombinant Antibody

The coding sequence for the light and heavy chain were recovered from the single B cells, which had been previously stored at -70°C. A two step reverse transcription polymerase chain reaction (RT-PCR) process was employed. In Step 1, the RNA encoding the areas of interest was recovered by a standard RT-based method that was subsequently amplified. Step 2 was conducted via a nested primer PCR amplification that generates the appropriate DNA fragments for directional cloning into the expression vector: Light chain: HindIII/BsiWI and Heavy chain: HindIII/XhoI. The specific sequences for this recovery process were derived from sequence analysis of the host animal genome. A major source of novel sequence is the rabbit, as well as the mouse and rat. The primer sequences were:

| | Primer SEQ ID NO. | Sequence (5' to 3') |
|---|---|---|
| Vk sense outer | 1 | AG[GA]ACCCAGC**ATG**GACA[CT][CGA]A |
| Vk sense inner | 2 | GATATCAAGCTTCGAATCGACATGGACACGAGGGCC CCC (HindIII/SfuI) |
| Ck anti-sense outer | 3 | GGA[TC][AG]G[AT]ATTTATT[CT]GCCAC[GA]CACA |
| Ck anti-sense inner | 4 | TCTAGACGTACGTTTGACCACCACCTCGGTCCCTC (BsiWI) |
| VH sense outer | 5 | AGAC[AG]CTCACC**ATG**GAGACT |
| VH sense inner | 6 | GATATCAAGCTTACGCTCACC**ATG**GAGACTGGGC (HindIII) |
| Cg CH1 anti-sense outer | 7 | ACTGGCTCCGGGAGGTA |
| Cg CH1 anti-sense inner | 8 | CGCGCGCTCGAGACGGTGACSAGGGTSCCYKGGCCCC (XhoI) |

Cloned cDNAs were then ligated into two distinct mammalian expression vectors (kappa light chain constant and gamma-1 (γ-1) heavy chain constant) that enable expression of the recombinant light and heavy chain. These constructs were made in frame and incorporated the natural signal sequence included in the sequence recovery. Large scale DNA preparations were made for each expression plasmid, and transient production of full length rabbit/human chimeric antibody was conducted by transfection using both plasmids into HEK293 cells. After 5 days in culture, the resulting cells were removed by centrifugation, and the condition medium was tested directly for antigen recognition, or the recombinant antibody was affinity purified via Protein A chromatography.

The antibody was then tested for antigen recognition using the ELISA method described above. In addition, for the purified antibody, the K_{d} was established by a ForteBio Octect measurement. Finally, the original function-modifying properties attributed to the particular well associated with the recovered sequence was tested.

### Experimental Method for Light and Heavy Chain Sequence Recovery.

The method is based on the technology described in the manufacturer's description for the Qiagen One Step RT-PCR kit. A common master mix was prepared and included RNasin (Promega) to prevent RNA degradation. 50 µL of RT-PCR master mix containing 0.58 µM of each step 1 primer (Primer SEQ ID NOs.: 1, 3, 5, and 7) was added to the 250 µL eppendorf tube containing previously recovered frozen cell and carefully mixed on ice. The One Step RT-PCR was performed with the following cycle scheme: (1) 50°C, 30 minutes; (2) 95°C, 15 minutes; (3) 94°C, 30 seconds; (4) 54°C, 30 seconds; (5) 72°C, 1 minute; (6) go to step 3, 35 cycles total; (7) 72°C, 3 minutes; and (8) 4°C, hold.

When these cycles were completed, the secondary PCR amplification was conducted in separate reactions to recover the light and heavy chain variable regions using 1.5 µL of the primary RT-PCR reaction. A KOD polymerase driven amplification (Novagen) with 0.4 µM of secondary nested PCR primers light chain (Primer SEQ ID NOs.: 2 and 4) and heavy chain (Primer SEQ ID NOs.: 6 and 8) using the following cycle scheme: (1) 94°C, 2 minutes; (2) 94°C, 30 seconds; (3) 60°C, 30 seconds; (4) 72°C, 45 seconds; (5) go to step 2, 35 cycles total; (6) 72°C, 3 minutes; and (7) 4°C, hold.

Upon completion of the secondary amplification, 10 µL of the reaction was removed and analyzed by 2% TAE agarose gel electrophoresis. The remaining 40 µL of the reaction were purified via Qiagen Qiaquick PCR Clean-up kit and eluted in 75 µL.

These amplicons were subsequently digested with HindIII/BsiWI in the case of light chain and HindIII/XhoI for the heavy chain using the following conditions: 10 µL Purified PCR product, 3 µL 10x New England Biolabs restriction enzyme buffer 2, 0.5 µL HindIII (5U), and 0.5 µL BsiWI (5U) or 0.5 uL XhoI for 60 minutes at 37°C followed by 30 minutes at 55°C. The digests were purified via Qiagen Qiaquick PCR method. These were subsequently ligated into the appropriate expression vector. 2 µL of this reaction was then used to transform either TOP10 (Invitrogen) or XL-10 (Stratagene), and the transformed cells were plated on LB/Kanamycin (50 µg/mL).

The resulting colonies were screened for inserts via a PCR screening method employing the following primers:

| | Primer SEQ ID NO. | Sequence (5' to 3') |
|---|---|---|
| Vector | 9 | GCGCGCCACCAGACATAATAGCT |
| Heavy Chain | 10 | AGCCCAAGGTCACCGTGCTAGAG |
| Light Chain | 11 | GTATTTATTCGCCACACACACACGATG |

Colonies were picked into 60 µL LB/kanamycin and incubated for up to 30 minutes. At 30 min, approximately 1 µL was removed and used in a standard 30 µL KOD amplification reaction (Novagen) containing 2 µM of the primer pair SEQ ID NOs.: 9/10 for the heavy chain and SEQ ID NOs.: 9/11 for the light chain. The amplification scheme was as follows: (1) 96°C, 2 minutes; (2) 96°C, 20 seconds; (3) 68°C, 25 seconds; (4) go to 2, repeat for 40 cycles total; and (5) 68°C, 2 minutes.

5 µL was removed and analyzed on 2% agarose. Following confirmation of a correct variable region insert, 5 µL of each reaction was digested with Alu1 (New England Biolabs) in New Biolabs restriction enzyme buffer 2 in 10 µL final volume and analyzed on 4% TAE agarose gel electrophoresis. A unique Alu pattern was identified from each well that is recovered. These were subsequently processed for sequence characterization.

## Claims

1. A method of identifying a B cell that secretes an antigen-specific antibody, the method comprising:
(a) harvesting B cells from an immunised rabbit host;
(b) enriching the harvested rabbit B cells to increase the proportion of B cells that are specific for said antigen by affinity purification of antigen-specific B cells using an antigen directly or indirectly attached to a solid matrix or support, thereby forming an enriched B cell population;
(c) culturing one or more sub-populations of cells from said enriched rabbit B cell population under conditions that favour the survival of a single B cell to produce a clonal population in at least one culture well;
(d) determining whether said cultured sub-populations produce an antibody specific to said antigen, thereby identifying one or more antigen-positive sub-populations; and
(e) determining whether a single rabbit B cell produces an antibody specific to the antigen by a method comprising:
(i) isolating one or more single rabbit B cells from one or more of said antigen-positive sub-populations of step (d);
(ii) providing an immobilized antigen comprising a matrix or solid support to which said antigen has been directly or indirectly attached;
(iii) incubating an individual rabbit B cell with said immobilized antigen;
(iv) incubating said immobilized antigen with a fluorophore labeled secondary antibody, wherein said secondary antibody is a host-specific anti-immunoglobulin antibody that binds antibodies of the host of step (a), thereby detecting whether an antibody secreted by said individual B cell is bound to said immobilized antigen; and
(v) identifying a B cell that secretes an antibody specific to said antigen by a fluorimetric assay that detects antibody-antigen complexes formed adjacent to the secreting cell.

2. The method of claim 1, wherein said immobilized antigen comprises antigen-loaded beads.

3. The method of claim 1, wherein step (a) comprises harvesting B cells from at least one source selected from the spleen, lymph nodes, bone marrow, and peripheral blood mononuclear cells and preferably pooling said B cells from said more than one source.

4. The method of claim 1, further comprising isolating or sequencing a nucleic acid encoding an antibody chain or fragment thereof from said individual B cell determined to produce an antibody specific to the antigen in step (e) and expressing a polypeptide encoded by said nucleic acid in a recombinant cell, preferably a yeast, bacterium, plant, insect, amphibian, or mammalian cell.

5. The method of claim 4, wherein said recombinant cell is a diploid yeast, preferably Pichia.

6. The method of claim 1, further comprising immunizing the host with the antigen prior to step (a).

7. The method of claim 6, wherein step (a) comprises harvesting B cells from the host at about 20 to about 90 days after said immunization or about 50 to about 60 days after said immunization.

8. The method of claim 1, wherein the host has been naturally exposed to the antigen prior to step (a).

9. The method of claim 1, wherein step (b) comprises affinity purification of antigen-specific B cells using an antigen directly or indirectly attached to a solid matrix or support being magnetic beads or a column.

10. The method of claim 9, wherein the antigen that is directly or indirectly attached to a solid matrix or support is biotinylated and is attached to the matrix or support via streptavidin, avidin, or neutravidin.

11. The method of claim 1, wherein the sub-populations of step (c) comprise no more than about 10,000 antigen-specific, antibody-secreting cells, or comprises about 50 to about 10,000 antigen-specific, antibody-secreting cells, or about 50 to about 5,000 antigen-specific, antibody-secreting cells., or about 50 to about 250 antigen-specific, antibody-secreting cells.

12. The method of claim 1, wherein the sub-populations of step (c) are cultured in a medium comprising feeder cells preferably EL4B cells.

13. The method of claim 1, wherein the sub-populations of step (c) are cultured in a medium comprising activated T cell conditioned medium, preferably a medium comprising between about 1% and about 5% activated rabbit T cell conditioned medium.

14. The method of claim 1, wherein the sub-populations of step (c) are cultured for at least 3 days, more preferably for between about 3 days and about 5 days or are cultured for at least one week.

15. The method of claim 1, further comprising determining whether said cultured sub-populations of step (c) produce an antibody that exhibits agonism or antagonism of antigen binding to a binding partner; induction or inhibition of the proliferation of a specific target cell type; induction or inhibition of lysis of a target cell; or induction or inhibition of a biological pathway involving the antigen.

16. The method of claim 1, further comprising determining the antigen binding affinity of an antibody produced by said cultured sub-populations of step (c).

## Patentansprüche

1. Verfahren zur Identifizierung von B-Zellen, welche einen Antigenspezifischen Antikörper sezernieren, umfassend:
(a) Gewinnen von B-Zellen aus einem immunisierten Kaninchen-Wirtstier;
(b) Anreichern der gewonnenen Kaninchen-B-Zellen, um den Anteil von für das Antigen spezifischen B-Zellen durch Affinitätsreinigung von Antigen-spezifischen B-Zellen unter Verwendung eines direkt oder indirekt an eine feste Matrix oder einen festen Träger gebundenen Antigens zu erhöhen, wobei eine angereicherte B-Zell-Population gebildet wird;
(c) Kultivieren einer oder mehrerer Subpopulationen von Zellen aus der angereicherten Kaninchen-B-Zell-Population unter Bedingungen, die das Überleben einer einzelnen B-Zelle fördern, um eine klonale Population in mindestens einer Kulturmulde zu erzeugen;
(d) Bestimmen, ob die kultivierten Subpopulationen einen für das Antigen spezifischen Antikörper produzieren, wodurch eine oder mehrere Antigen-positive Subpopulationen identifiziert werden; und
(e) Feststellen, ob eine einzelne Kaninchen-B-Zelle einen gegen das Antigen spezifischen Antikörper produziert durch ein Verfahren, welches umfasst:
(i) Isolieren einer oder mehrerer einzelner Kaninchen-B-Zellen aus einer oder mehreren der Antigen-positiven Subpopulationen aus Schritt (d);
(ii) Bereitstellen eines immobilisierten Antigens umfassend eine Matrix oder einen festen Träger, an die oder den das Antigen direkt oder indirekt befestigt wurde;
(iii) Inkubieren einer einzelnen Kaninchen-B-Zelle mit dem immobilisierten Antigen;
(iv) Inkubieren des immobilisierten Antigens mit einem durch ein Fluorophor markierten sekundären Antikörper, wobei der sekundäre Antikörper ein wirtsspezifischer Anti-Immunglobulin-Antikörper ist, der Antikörper des Wirtstiers aus Schritt (a) bindet, wodurch bestimmt wird, ob ein durch die einzelne B-Zelle sezernierter Antikörper an das immobilisierte Antigen gebunden ist; und
(v) Identifizieren einer B-Zelle, welche einen gegen das Antigen spezifischen Antikörper sezerniert, durch einen fluorimetrischen Test, der Antikörper-Antigenkomplexe erkennt, welche benachbart zu der sezernierenden Zelle gebildet wurden.

2. Verfahren nach Anspruch 1, worin das immobilisierte Antigen Antigenbeladene Beads umfasst.

3. Verfahren nach Anspruch 1, worin Schritt (a) das Gewinnen von B-Zellen aus mindestens einer Quelle umfasst, welche ausgewählt ist aus Milz, Lymphknoten, Knochenmark und peripheren mononukleären Blutzellen und vorzugsweise Vereinigen der B-Zellen aus der mehr als einen Quelle umfasst.

4. Verfahren nach Anspruch 1, welches weiter umfasst das Isolieren oder Sequenzieren einer eine Antikörperkette oder ein Fragment hiervon kodierenden Nukleinsäure aus der einzelnen B-Zelle, für welche in Schritt (e) festgestellt wurde, dass sie einen für das Antigen spezifischen Antikörper produziert, und Exprimieren eines Polypeptids, welches durch die Nukleinsäure kodiert wird in einer rekombinanten Zelle, vorzugweise einer Hefe-, bakteriellen, Pflanzen-, Insekten-, Amphibienoder Säuger-Zelle.

5. Verfahren nach Anspruch 4, worin die rekombinante Zelle eine diploide Hefezelle, vorzugsweise Pichia, ist.

6. Verfahren nach Anspruch 1, welches außerdem vor Schritt (a) das Immunisieren des Wirtstiers mit dem Antigen umfasst.

7. Verfahren nach Anspruch 6, worin Schritt (a) das Gewinnen von B-Zellen aus dem Wirtstier zum Zeitpunkt von ungefähr 20 bis ungefähr 90 Tagen nach der Immunisierung oder ungefähr 50 bis ungefähr 60 Tagen nach der Immunisierung umfasst.

8. Verfahren nach Anspruch 1, worin das Wirtstier vor Schritt (a) dem Antigen auf natürliche Weise exponiert wurde.

9. Verfahren nach Anspruch 1, worin Schritt (b) eine Affinitätsreinigung von Antigen-spezifischen B-Zellen unter Verwendung von direkt oder indirekt an magnetische Beads oder eine Säule als fester Matrix oder festem Träger befestigtem Antigen umfasst.

10. Verfahren nach Anspruch 9, worin das Antigen, welches direkt oder indirekt an eine feste Matrix oder einen Träger befestigt ist, biotinyliert und über Streptavidin, Avidin oder Neutravidin an die Matrix oder den Träger gebunden ist.

11. Verfahren nach Anspruch 1, worin die Subpopulationen aus Schritt (c) nicht mehr als ungefähr 10.000 Antigen-spezifische, Antikörpersezernierende Zellen oder ungefähr 50 bis ungefähr 10.000 Antigenspezifische, Antikörper-sezernierende Zellen oder ungefähr 50 bis ungefähr 5.000 Antigen-spezifische, Antikörper-sezernierende Zellen, oder ungefähr 50 bis ungefähr 250 Antigen-spezifische, Antikörpersezernierende Zellen umfassen.

12. Verfahren nach Anspruch 1, worin die Subpopulationen aus Schritt (c) in einem Medium, welches Fütterzellen, bevorzugt EL4B-Zellen, umfasst, kultiviert werden.

13. Verfahren nach Anspruch 1, worin die Subpopulationen aus Schritt (c) in einem Medium kultiviert werden, welches durch aktivierte T-Zellen konditioniertes Medium, vorzugsweise ein durch zwischen ungefähr 1% und ungefähr 5% aktivierte Kaninchen-T-Zellen konditioniertes Medium, umfasst.

14. Verfahren nach Anspruch 1, worin die Subpopulationen aus Schritt (c) für mindestens drei Tage, mehr bevorzugt zwischen ungefähr drei Tagen und ungefähr fünf Tagen, oder für mindestens eine Woche kultiviert werden.

15. Verfahren nach Anspruch 1, weiterhin umfassend das Feststellen, ob die kultivierten Subpopulationen aus Schritt (c) einen Antikörper produzieren, der Agonismus oder Antagonismus für Antigen-Bindung an einen Bindungspartner; Induktion oder Inhibition der Proliferation eines spezifischen Zielzelltypus; Induktion oder Inhibition der Lyse einer Zielzelle; oder Induktion oder Inhibition eines biologischen Reaktionsweges, an dem das Antigen beteiligt ist, zeigt.

16. Verfahren nach Anspruch 1, weiterhin umfassend die Bestimmung der Antigen-Bindungsaffinität eines Antikörpers, der durch die kultivierte Subpopulation von Schritt (c) erzeugt wird.

## Revendications

1. Procédé d'identification d'une cellule B qui sécrète un anticorps spécifique à un antigène, le procédé comprenant le fait :
(a) de collecter des cellules B à partir d'un lapin hôte immunisé ;
(b) d'enrichir les cellules B de lapin collectées pour augmenter la proportion de cellules B qui sont spécifiques audit antigène par purification par affinité de cellules B spécifiques à un antigène en utilisant un antigène directement ou indirectement fixé à un support ou une matrice solide, formant ainsi une population de cellules B enrichies ;
(c) de mettre en culture une ou plusieurs sous-population(s) de cellules provenant de ladite population de cellules B de lapin enrichies dans des conditions qui favorisent la survie d'une cellule B unique pour produire une population clonale dans au moins un puits de culture ;
(d) de déterminer si lesdites sous-populations mises en culture produisent un anticorps spécifique audit antigène, identifiant ainsi une ou plusieurs sous-population(s) positive(s) à l'égard de l'antigène ; et
(e) de déterminer si une cellule B unique de lapin produit un anticorps spécifique à l'antigène par un procédé comprenant le fait :
(i) d'isoler une ou plusieurs cellule(s) B unique(s) de lapin à partir d'une ou de plusieurs sous-population(s) parmi lesdites sous-populations positives à l'égard de l'antigène de l'étape (d) ;
(ii) de fournir un antigène immobilisé comprenant une matrice ou un support solide à laquelle/auquel ledit antigène a été fixé directement ou indirectement ;
(iii) d'incuber une cellule B individuelle de lapin avec ledit antigène immobilisé ;
(iv) d'incuber ledit antigène immobilisé avec un anticorps secondaire marqué avec un fluorophore, où ledit anticorps secondaire est un anticorps anti-immunoglobuline spécifique à l'hôte qui se lie à des anticorps de l'hôte de l'étape (a), détectant ainsi si un anticorps sécrété par ladite cellule B individuelle est lié audit antigène immobilisé ; et
(v) d'identifier une cellule B qui sécrète un anticorps spécifique audit antigène par un test fluorimétrique qui détecte des complexes anticorps-antigène formés de manière adjacente à la cellule sécrétrice.

2. Procédé de la revendication 1, dans lequel ledit antigène immobilisé comprend des billes chargées en antigène.

3. Procédé de la revendication 1, dans lequel l'étape (a) comprend le fait de collecter des cellules B à partir d'au moins une source choisie entre la rate, les ganglions lymphatiques, la moelle osseuse et des cellules mononucléées du sang périphérique et de regrouper, de préférence, lesdites cellules B provenant de ladite plus d'une source.

4. Procédé de la revendication 1, comprenant en outre le fait d'isoler ou de séquencer un acide nucléique codant pour une chaîne d'anticorps ou un fragment de celle-ci à partir de ladite cellule B individuelle déterminée comme produisant un anticorps spécifique à l'antigène dans l'étape (e), et d'exprimer un polypeptide codé par ledit acide nucléique dans une cellule recombinante, de préférence une cellule de levure, de bactérie, de plante, d'insecte, d'amphibien, ou de mammifère.

5. Procédé de la revendication 4, dans lequel ladite cellule recombinante est une levure diploïde, de préférence Pichia.

6. Procédé de la revendication 1, comprenant en outre le fait d'immuniser l'hôte avec l'antigène avant l'étape (a).

7. Procédé de la revendication 6, dans lequel l'étape (a) comprend le fait de collecter des cellules B provenant de l'hôte environ 20 à environ 90 jours après ladite immunisation ou environ 50 à environ 60 jours après ladite immunisation.

8. Procédé de la revendication 1, dans lequel l'hôte a été naturellement exposé à l'antigène avant l'étape (a).

9. Procédé de la revendication 1, dans lequel l'étape (b) comprend une purification par affinité des cellules B spécifiques à l'antigène en utilisant un antigène directement ou indirectement fixé à une matrice ou un support solide qui est des billes magnétiques ou une colonne.

10. Procédé de la revendication 9, dans lequel l'antigène qui est directement ou indirectement fixé à une matrice ou un support solide est biotinylé et est fixé à la matrice ou au support par l'intermédiaire de la streptavidine, l'avidine ou la neutravidine.

11. Procédé de la revendication 1, dans lequel les sous-populations de l'étape (c) comprennent pas plus d'environ 10 000 cellules sécrétrices d'anticorps spécifiques à l'antigène, ou comprennent environ 50 à environ 10 000 cellules sécrétrices d'anticorps spécifiques à l'antigène, ou environ 50 à environ 5000 cellules sécrétrices d'anticorps spécifiques à l'antigène, ou environ 50 à environ 250 cellules sécrétrices d'anticorps spécifiques à l'antigène.

12. Procédé de la revendication 1, dans lequel les sous-populations de l'étape (c) sont mises en culture dans un milieu comprenant des cellules nourricières de préférence des cellules EL4B.

13. Procédé de la revendication 1, dans lequel les sous-populations de l'étape (c) sont mises en culture dans un milieu comprenant un milieu conditionné par des cellules T activées, de préférence un milieu comprenant entre environ 1 % et environ 5 % de milieu conditionné par des cellules T activées de lapin.

14. Procédé de la revendication 1, dans lequel les sous-populations de l'étape (c) sont mises en culture pendant au moins 3 jours, plus préférablement pendant environ 3 jours à environ 5 jours ou sont mises en culture pendant au moins une semaine.

15. Procédé de la revendication 1, comprenant en outre le fait de déterminer si lesdites sous-populations mises en culture de l'étape (c) produisent un anticorps qui présente l'agonisme ou l'antagonisme d'un antigène se liant à un partenaire de liaison ; l'induction ou l'inhibition de la prolifération d'un type de cellule cible spécifique ; l'induction ou l'inhibition de la lyse d'une cellule cible ; ou l'induction ou l'inhibition d'une voie biologique impliquant l'antigène.

16. Procédé de la revendication 1, comprenant en outre le fait de déterminer l'affinité de liaison à l'antigène d'un anticorps produit par lesdites sous-populations mises en culture de l'étape (c).
